# EUROPEAN PATENT SPECIFICATION

(11) **EP 2 090 272 B1**
(45) Date of publication and mention of the grant of the patent: **12.05.2010**
(21) Application number: 08019134.9
(22) Date of filing: 31.10.2008
(51) Int. Cl.: A61F 11/14

(54) **Earmuff device**
Ohrschützervorrichtung
Dispositif de protège-oreilles

(30) Priority: 15.02.2008 TW 97105463
(43) Date of publication of application: 19.08.2009
(73) Proprietor: Sound Team Enterprise Co., Ltd., Pei-Tou Dist. Taipei City (TW)
(72) Inventor: Chiang, Tsung-Pai, Pei-Tou Dist. Taipei City (TW)
(74) Representative: Glawe, Delfs, Moll

(56) References cited:
- WO-A-02/053061
- JP-A- 2002 011 036
- US-A- 5 835 609
- US-A1- 2004 216 946

## Description

### BACKGROUND OF THE INVENTION

### 1. Field of the Invention:

The present invention relates to earmuffs and more particularly, to an earmuff device that allows the wearer to conveniently adjust the length of the headpiece as well as the angular position of the headpiece relative to the earmuff cups. Further, the earmuff device can be received in a collapsed manner with the two earmuff cups attached together to save storage space.

### 2. Description of the Related Art:

In winter or cold places, people usually wear heavy clothes, muffler and gloves to keep the body and the hands warm. When walking in a high mountain, snow land and outdoors during a winter season, regular clothes cannot well protect the ears, and the ears may ache or may be affected by frostbite. For keeping the ears warm, earmuffs shall be used.

FIG. 16 illustrates a conventional earmuff device (US 5,835,609; Fig. 26).
According to this design, the earmuff device comprises an adjustable headpiece **A** that is formed of two bands axially slidably coupled to each other, and two earmuffs **B** respectively fastened to the two distal ends of the headpiece **A** with a respective fastening member **C**. When in use, the headpiece **A** is adjusted to the desired length, and then the two earmuffs **B** are respectively attached to the ears. This design of earmuff device is still not satisfactory in function because of the following drawbacks:
1. The earmuff device can be worn on the user's head from the top or back side in one single direction.
2. The protruding ring end of each band of the headpiece A is kept in contact with the wearer's head, causing the wearer to feel uncomfortable.
3. Because the two bands of the headpiece **A** have a uniform transverse thickness, the headpiece **A** may be twisted easily. Further, because the angular position of the earmuffs **B** is not adjustable relative to the headpiece **A,** the earmuff device may fall from the wearer's head easily by an external force.
4. Because the earmuff device simply utilizes the small fastening member **C** to secure the earmuffs **B** to the headpiece **A,** the connection area between the earmuffs **B** and the headpiece **A** may break easily when the wearer adjust the position of the earmuffs **B.**
5. The earmuffs **B** have low structural strength and low grasping force. In consequence, the earmuffs **B** cannot cover the wearer's ears positively.
6. When the earmuff device is not in use, the user cannot receive the earmuff device in a collapsed condition with the earmuffs **B** attached together to save storage space. When receiving the earmuff device, the earmuff device may be broken accidentally.
7. The earmuff device is packed in the assembled condition. Therefore, this design does not allow the headpiece and the earmuffs to be separately wrapped with a respective fabric covering.

### SUMMARY OF THE INVENTION

The present invention has been accomplished to provide an earmuff device that eliminates the aforesaid drawbacks. According to one aspect of the present invention, the earmuff device is comprised of a headpiece, two earmuff cups, two connecting devices respectively connecting the earmuff cups to the headpiece, and two earmuff cushions respectively covered on the earmuff cups. The headpiece is comprised of two smoothly arched bands that are overlapped on each other and axially slidable relative to each other. Each band has a coupling block disposed at one end and defining an insertion slot, and two sliding grooves bilaterally extending along the length. One band is inserted through the insertion slot of the other band and the coupling block of one band is slidably coupled to the sliding grooves of the other band so that the two bands are kept in a parallel manner and can be moved relative to each other to adjust the length of the headpiece. Because the sliding grooves bilaterally extend along the length of the respective band, the two opposite lateral sides of each band are relatively thinner. Therefore, the stress is concentrated on the middle part of each band during a use of the earmuff device, preventing distortion of the earmuff device.

According to another aspect of the present invention, each connecting device comprises an U-lug pivotally coupled to one end of one band of the headpiece, and a connecting strip pivoted to the U-lug and fastened to one earmuff cup. Therefore, the user can adjust the earmuff cups forwards and backwards as well as leftwards and rightwards. Further, each band has a chamfered end edge kept in friction engagement with the associating U-lug so that the U-lug can be positively positioned in one angular position relative to the associating band.

According to another aspect of the present invention, each earmuff cup has a cup base, an insertion hole in the cup base, and at least one engagement hole cut through the cup base across the insertion hole. Further, the connecting strip of each connecting device is inserted into the insertion hole of one earmuff cup, having at least one retaining rib respectively forced into engagement with the at least one engagement hole of the associating earmuff cup.

According to still another aspect of the present invention, the earmuff cushions are respectively made in the form of a pocket member that receives the associating earmuff cup. Further, each earmuff cushion has an opening through which the connecting strip of the associating connecting device is inserted into the insertion hole of the associating earmuff cup. Further, a flexible fabric sleeve may be sleeved onto the headpiece and connected between the earmuff cushions.

According to still another aspect of the present invention, the U-lugs of the connecting devices can be turned relative to the headpiece and the earmuff cups can be turned with the associating connecting strips relative to the associating U-lugs so that the earmuff device can be received in a compact condition, saving the storage space.

### BRIEF DESCRIPTION OF THE DRAWINGS

FIG. 1 is a perspective view of an earmuff device according to the present invention (the earmuff cushions excluded).
FIG. 2 is an exploded view of FIG. 1.
FIG. 3 is a schematic drawing showing adjustment of the length of the headpiece according to the present invention.
FIG. 4 is a sectional view of a part of the present invention, showing connection between one band of the headpiece and the associating connecting device.
FIG. 5 is a sectional view of a part of the present invention, showing connection of one connecting device between the associating band and the associating earmuff cup.
FIG. 6 is a schematic drawing showing the earmuff cup turned with the associating connecting device relative to the headpiece according to the present invention.
FIG. 7 is a schematic sectional view of a part of the present invention, showing the earmuff cup turned with the connecting strip of the associating connecting device relative to the associating U-lug and the headpiece.
FIG. 8 illustrates one received status of the earmuff device according to the present invention (the earmuff cushions excluded).
FIG. 9 illustrates another received status of the earmuff device according to the present invention (the earmuff cushions excluded).
FIG. 10 is an exploded view of a part of the present invention, showing one form of the earmuff cushion.
FIG. 11 is a sectional view of a part of the present invention, showing another form of the earmuff cushion.
FIG. 12 is a perspective view of the present invention, showing the two earmuff cushions respectively covered on the earmuff cups.
FIG. 13 is a perspective view of an alternate form of the present invention, showing a fabric sleeve sleeved onto the headpiece and connected between the earmuff cushions.
FIG. 14 is a perspective view of another alternate form of the present invention, wherein the fabric sleeve is formed integral with the earmuff cushions.
FIG. 15 is a schematic drawing showing wearing adjustment of the earmuff device according to the present invention.

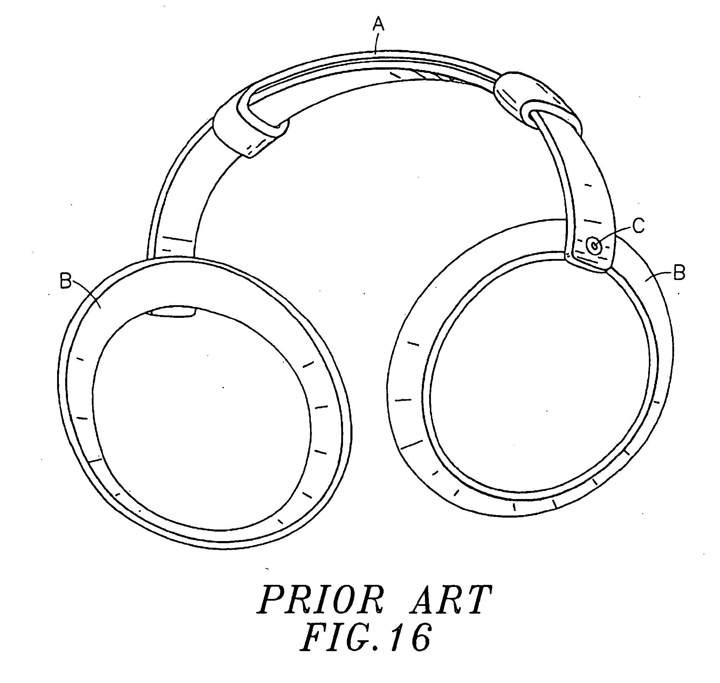

FIG. 16 is an elevational view of an earmuff device according to the prior art.

### DETAILED DESCRIPTION OF THE PREFERRED EMBODIMENT

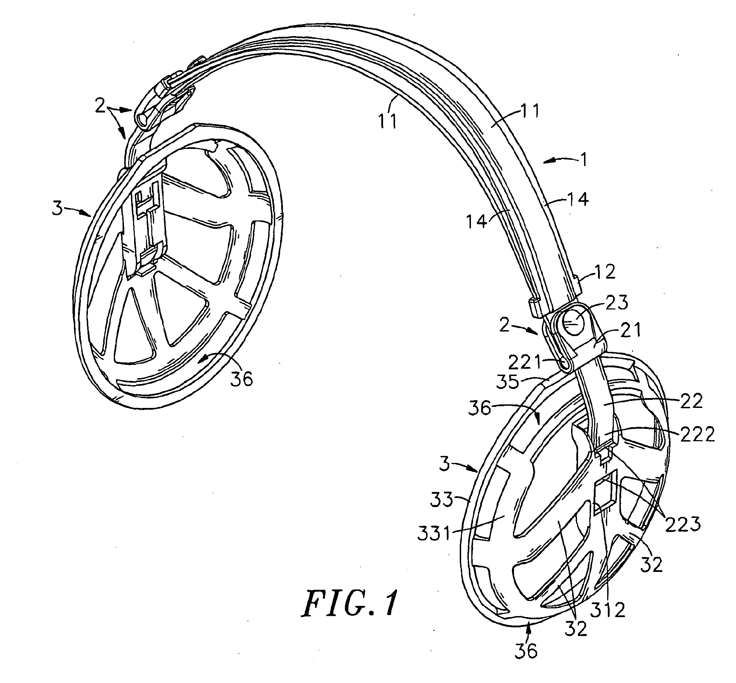

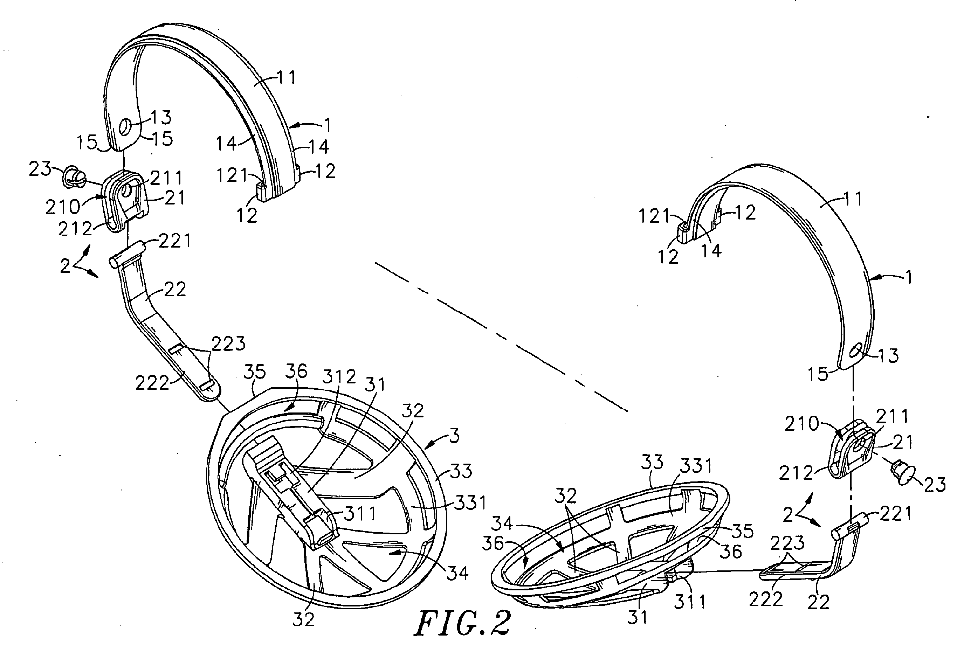

Referring to FIGS. 1, 2, 10 and 11, an earmuff device is shown comprising a headpiece **1,** two connecting devices **2,** two earmuff cups **3** and two earmuff cushions **4.**

The headpiece **1** is comprised of two smoothly arched bands **11** that are overlapped on each other and axially slidable relative to each other. Each band **11** has a first end provided with a chamfered end edge **15** and provided with a through hole **13,** a second end opposing to the first end and provided with a coupling block **12** that defines an insertion slot **121,** and two sliding grooves **14** bilaterally extending along the length between the first end and the second end. By means of inserting the first end of one band **11** through the insertion slot **121** of the coupling block **12** of the other band **11,** the two bands **11** are coupled together and axially slidable relative to each other to adjust the combined length, and the sliding grooves **14** of one band **11** guide sliding movement of the coupling block **12** of the other band **11.**

The connecting devices **2** are adapted for securing the two earmuff cups **3** to the first ends of the two bands **11** of the headpiece **1** respectively, each comprised of a U-lug **21,** a connecting strip **22** and a pivot bolt **23.** The U-lug **21** has a flat receiving open chamber **210** defined between the two opposite sidewalls thereof, two through holes **211** respectively cut through the two opposite sidewalls and aligned in line at two opposite sides of the flat receiving open chamber **210,** and a bottom pivot housing **212** connected between the two opposite sidewalls. The connecting strip **22** has a pivot axle **221** transversely disposed at its one end and pivotally coupled to the bottom pivot housing **212** of the U-lug **21,** a connecting endpiece **222** disposed at its other end, and at least one, for example, two retaining ribs **223** transversely disposed at the connecting endpiece **222** and spaced in direction along the length of the connecting endpiece **222.**

The earmuff cups **3** are respectively fastened to the connecting strips **22** of the connecting devices **2,** each having a cup base **31,** an insertion hole **311** defined in the cup base **31** for the insertion of the connecting strip **22** of the associating connecting device **2,** at least one, for example, two engagement holes **312** cut through the cup base **31** and extending across the insertion hole **311,** a gradual enlargement cup rim **33,** a plurality of radial ribs **32** radially connected between the cup base **31** and gradual enlargement the cup rim **33,** a plurality of smoothly arched reinforcing ribs **331** connected with the radial ribs **32,** an accommodation space **34** surrounded by the radial ribs **32,** the cup base **31,** the cup rim **33** and the smoothly arched reinforcing ribs **331,** a plurality of locating holes **36** defined between the cup rim **33** and the smoothly arched reinforcing ribs **331** and separated by the radial ribs **32.**

When the headpiece **1** and the earmuff cups **3** are fastened together by the connecting devices **2,** the two earmuff cushions 4 are respectively covered on the two earmuff cups **3** for contact with the side of the wearer's head.

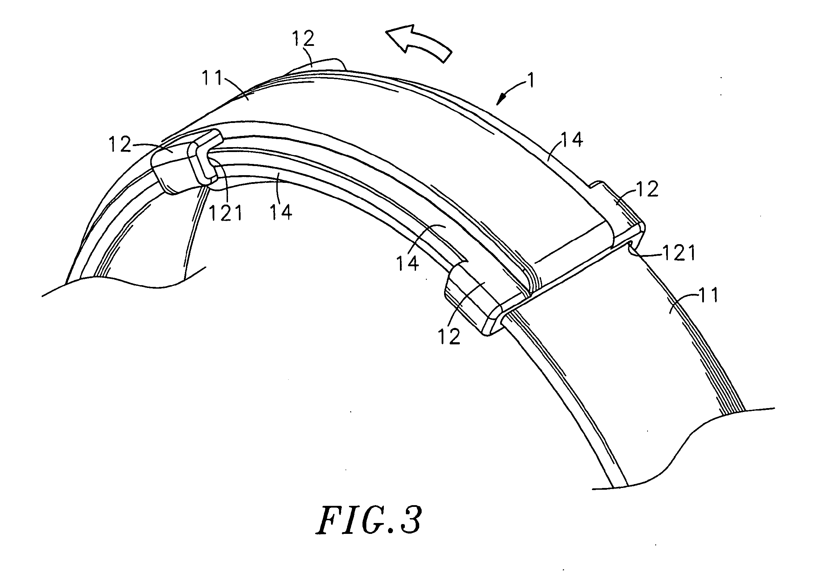

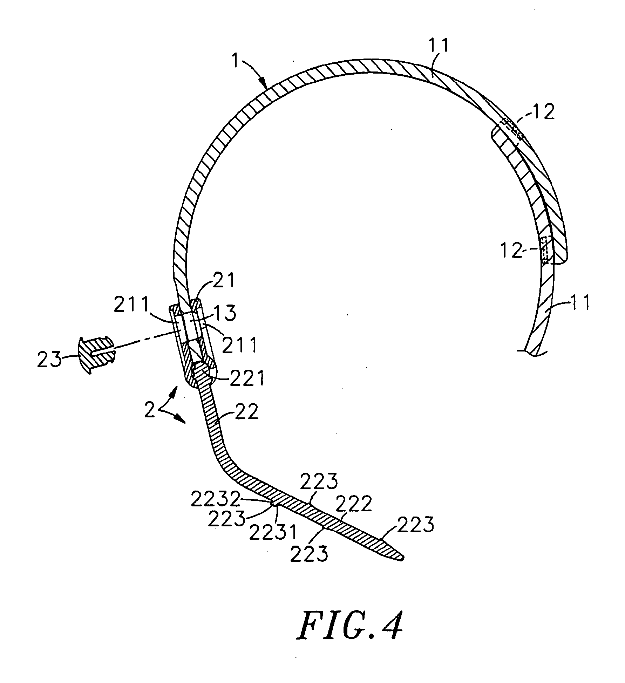

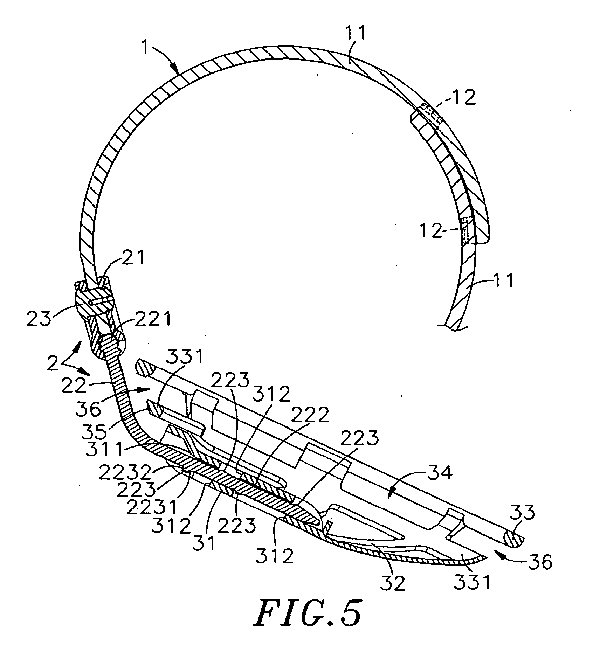

Referring to FIGS. 3∼5, the first end of one band **11** is inserted through the insertion slot **121** of the coupling block **12** of the other band **11** so that the two bands **11** are coupled together and can be moved axially relative to each other to adjust the combined length (see FIG. 3). When adjusting the length of the headpiece **1,** the sliding grooves **14** of one band **11** guide sliding movement of the coupling block **12** of the other band **11.** Thereafter, the pivot axles **221** of the connecting strips **22** of the connecting devices **2** are respectively pivotally coupled to the bottom pivot housings **212** of the associating U-lugs **21,** and then the bands **11** are respectively inserted into the flat receiving open chambers **210** of the U-lugs **21** to have the respective through holes **13** of the bands **11** be in alignment with the respective through holes **211** of the U-lugs **21,** and then the pivot bolts **23** are respectively fastened to the through holes **211** and **13** to pivotally secure the U-lugs **21** to the bands **11** of the headpiece **1** (see FIG. 4). Thereafter, the connection endpieces **222** of the connecting strips **22** of the connecting devices **2** are respectively inserted into the insertion holes **311** of the earmuff cups **3** to force the retaining ribs **223** of the respective connecting strips **22** into engagement with the respective engagement holes **312** of the earmuff cups **3** (see FIG. 5).

Further, the cross section of each retaining rib **223** is a regular triangle, having a front guide slope **2231** and a rear upright stop edge **2232.** By means of the front guide slopes **2231,** the retaining ribs **223** can conveniently be forced into the engagement holes **312.** After insertion of the retaining ribs **223** into the associating engagement holes **312,** the rear upright stop edge **2232** of each retaining rib **223** is stopped against one side of the associating engagement hole **312** to prohibit backward displacement of the respective retaining rib **223** relative to the associating engagement hole **312,** and therefore the earmuff cups **3** are respectively and positively secured to the connecting strips **22** of the connecting devices **2.**

Referring to FIGS. 1~3 again, when the two bands **11** are coupled together, the user can move the two bands **11** relative to each other to adjust the length of the headpiece **1.** When adjusting the length of the headpiece **1,** the sliding grooves **14** of one band **11** guide sliding movement of the coupling block **12** of the other band **11.** Further, each band **11** has a thickness gradually reducing in direction from the coupling block **12** toward the through hole **13,** i.e., from the second end toward the first end. This band thickness design enables the headpiece **1** to be comfortably attached to the wearer's head. Further, by means of matching the recessed sliding grooves **14** of one band **11** with the protruding coupling block **12** of the other band **11,** the two bands **11** are matched smoothly in a flat manner.

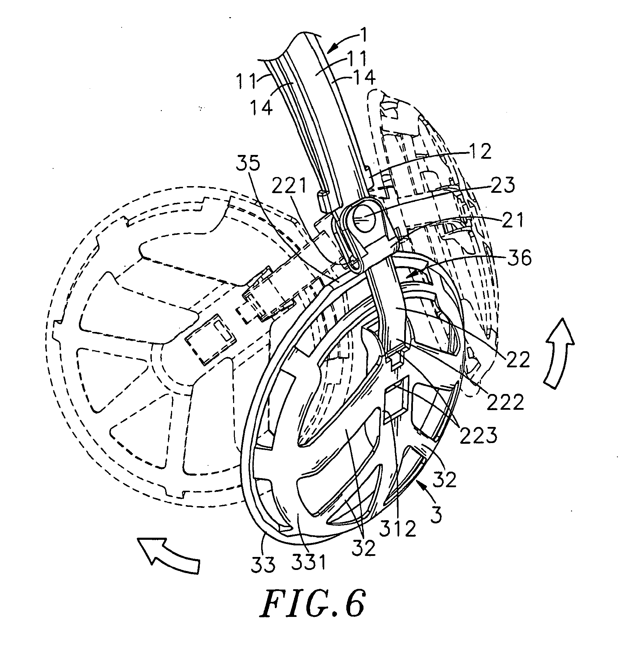

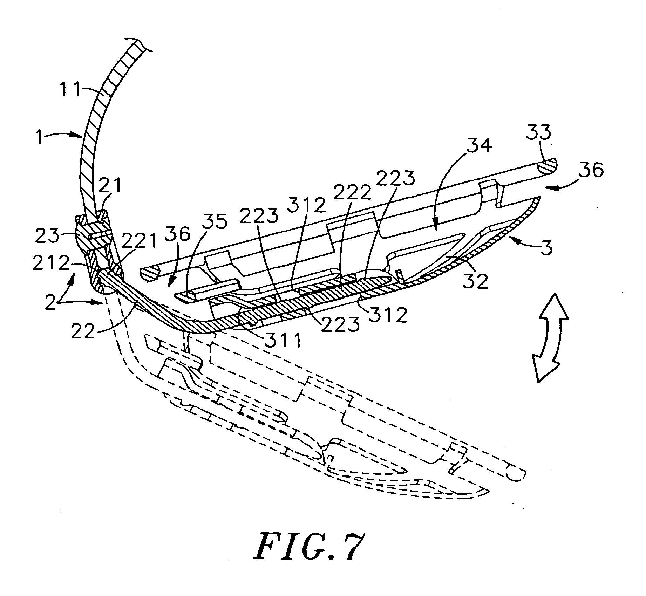

Referring to FIGS. 6 and 7, the U-lugs **21** of the connecting devices **2** are respectively pivotally connected to the through holes **13** of the bands **11** by the respective pivot bolts **23,** and therefore the earmuff cups **3** can be biased with the connecting devices **2** forwards and backwards relative to the headpiece **1.** When biasing the earmuff cups **3** with the connecting devices **2** forwards and backwards relative to the headpiece **1,** the chamfered end edge **15** of the first end of each band **11** is rubbed against the associating U-lug **21.** After adjustment, the chamfered end edge **15** of the first end of each band **11** is stopped against the inside wall of the associating U-lug **21,** stopping the earmuff cups **3** in one of three positions, the middle position in line with the headpiece **1,** namely, the front position perpendicular to the headpiece **1,** and the rear position perpendicular to the headpiece **1** (see FIG. 6). Further, the coupling between the pivot axle **221** of the connecting strip **22** of each connecting device **2** and the bottom pivot housing **212** of the respective U-lug **21** allows the earmuff cups **3** to be biased with the associating connecting strips **22** relative to the associating U-lugs **21** and the headpiece **1** in direction away from or toward the wearer's head (see FIG. 7). Therefore, the wearer can adjust the earmuff device to fit the head comfortably.

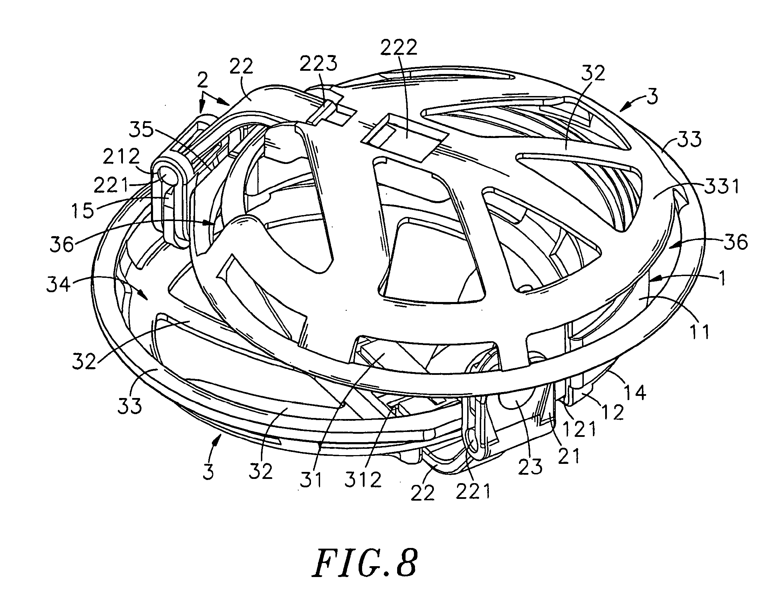

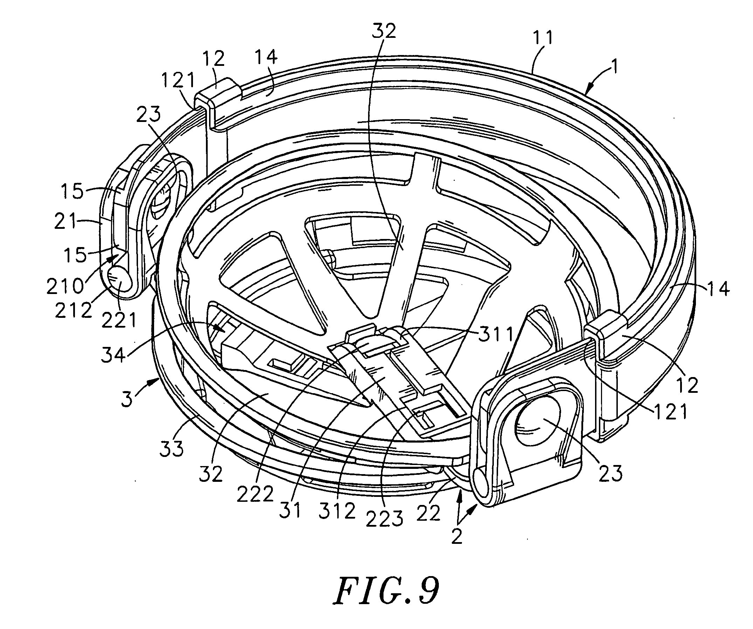

Referring to FIGS. 8 and 9, when not in use, the two bands **11** are moved toward each other to shorten the length of the headpiece **1,** and then the connecting device **2** are turned to adjust the earmuff cups **3** relative to the headpiece **1** and closely attached to each other in reversed directions (see FIG. 8). Alternatively, the connecting device **2** are turned to adjust the earmuff cups **3** relative to the headpiece **1** and closely attached to each other in one same direction (see FIG. 9). Therefore, the earmuff device can be adjusted to fit the wearer's head comfortably. When not in use, the earmuff device can be collapsed and received in a compact condition, saving much the storage space.

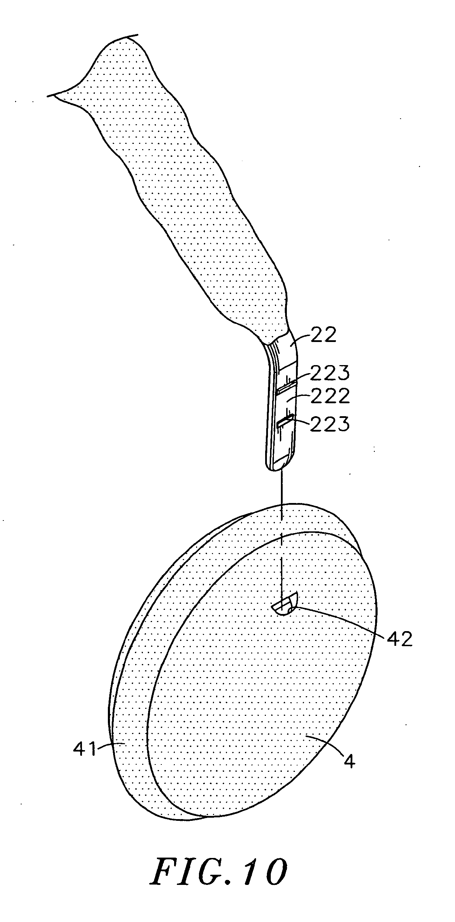

Referring to FIG. 10 and FIG. 1 again, each earmuff cushion **4** covers one earmuff cup **3** and a part of the connecting strip **22** of the associating connecting device **2** for contact with the side of the wearer's head. Further, the bands **11** of the headpiece **1** and the connecting devices **2** may be respectively covered with soft covering means. According to the embodiment shown in FIG. 10, the earmuff cushion **4** is comprised of two fabric panels respectively attached to the front and back sides of the earmuff cup **3** and a binding strip **41** stitched to the two fabric panels. Further, the earmuff cushion **4** has an opening **42** corresponding to the insertion hole **311** of the associating earmuff cup **3** for the insertion of the associating connecting strip **22.**

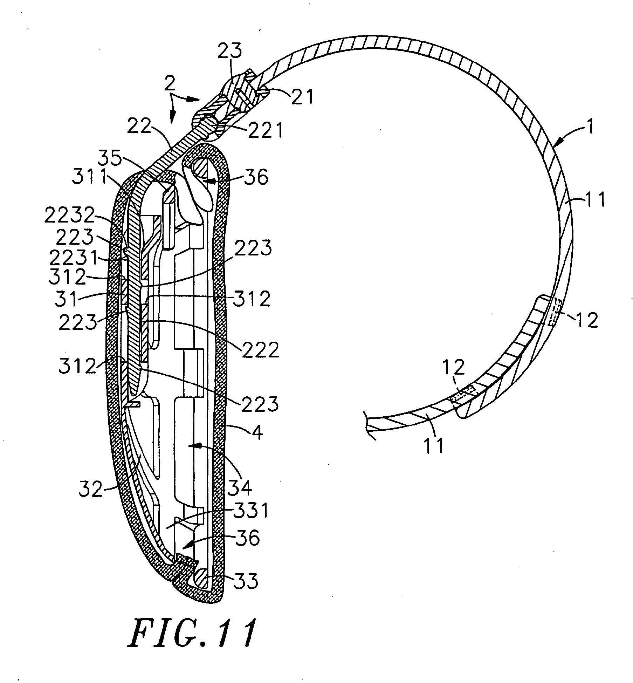

FIG. 11 shows an alternate form of the earmuff cushion **4.** According to this embodiment, the earmuff cushion **4** is made in the form of a fabric pocket for receiving one earmuff cup **3.** The fabric pocket can be formed by means of stitching two fabric panels together, and then turning the fabric pocket inside out to have the earmuff cup 3 be received inside the fabric pocket and also to have the stitches be kept from sight. After installation, the cuff around the opening of the fabric pocket is turned inwards toward the inside of the fabric pocket and inserted into one locating hole **36** of the earmuff cup **3.**

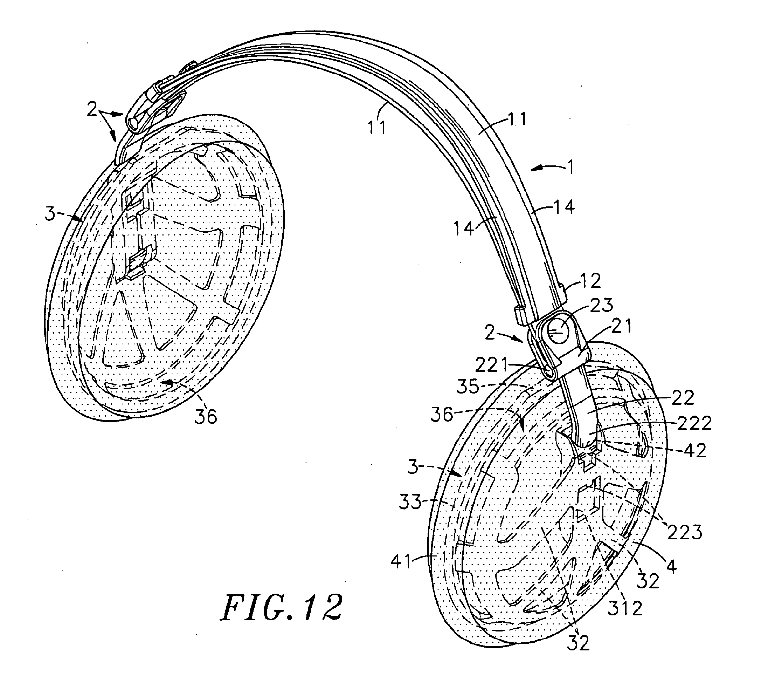

FIG. 12 is a perspective view of the present invention, showing the earmuff cushions **4** respectively covered on the earmuff cups **3,** and the connecting strips **22** of the connecting devices **2** are respectively inserted into the openings **42** of the earmuff cushions **4** and connected to the associating earmuff cups **3.**

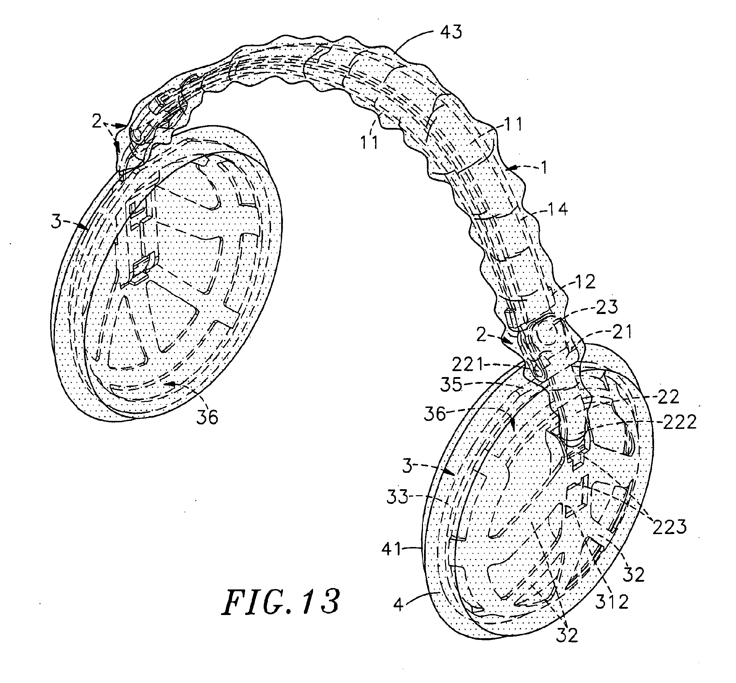

Referring to FIG. 13, a fabric sleeve **43** may be sleeved onto the headpiece **1** and connected between the two earmuff cushions **4.** The fabric sleeve **43** has a certain length that allows adjustment of the length of the headpiece **1** to a certain extent.

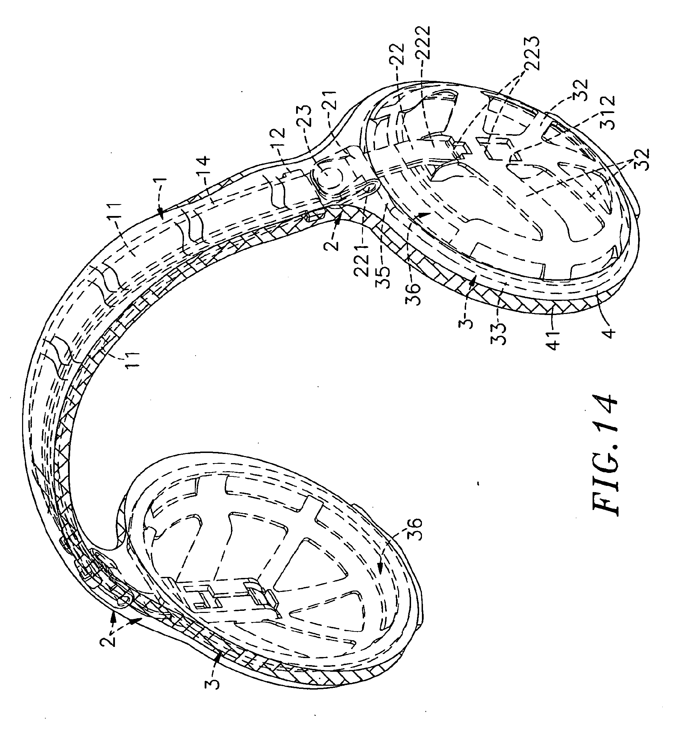

FIG. 14 shows still another alternate form of the present invention. This embodiment is substantially similar to the embodiment shown in FIG. 13 with the exception that the fabric sleeve **43** and the earmuff cushions **4** are made in integrity, i.e., two fabric panels are attached to the headpiece **1,** the connecting devices **2** and the earmuff cups **3** from two opposite sides and then peripherally stitched together. Alternatively, a fabric material can be used to make a pocket subject to the configuration of the earmuff device, and then the earmuff device is inserted into the pocket, and then the cuff around the opening of the pocket is received together and inserted into one locating hole 36 of one earmuff cup **3.**

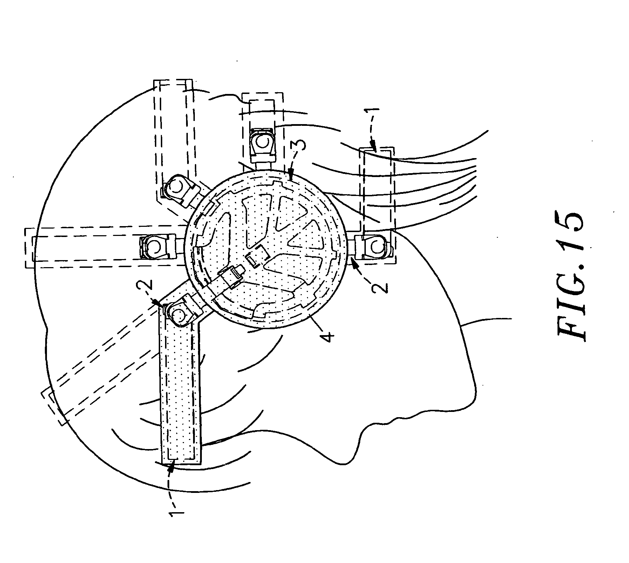

Referring to FIG. 15 and FIG. 1, when a person is wearing the earmuff device of the present invention, the wearer can adjust angular position of the headpiece **1** relative to the earmuff cups **3**. For example, the headpiece **1** can be attached to the wearer's forehead, the top or back of the wearer's head or the back of the wearer's neck, while the earmuff cups **3** are attached to the wearer's ears, i.e., the user can freely adjust the length of the headpiece **1** and the angle of the earmuff cups **3** relative to the headpiece **1** as desired when wearing the earmuff device.

Although particular embodiments of the invention have been described in detail for purposes of illustration, various modifications and enhancements may be made without departing from the scope of the invention.
- 1: Headpiece
11. Band
12. Coupling hole
121. Insertion slot
13. Through hole
14. Sliding groove
15. Chamfered end edge
- 2.: Connecting device
21. U-lug
210. Open chamber
211. Through hole
212. Bottom pivot housing
22. Connecting strip
221. Pivot axle
222. Connecting endpiece
223. Retaining rib
2231. Front guide slope
2232. Rear upright stop edge
23. Pivot bolt
- 3.: Earmuff cup
31. Cup base
311. Insertion hole
312. Engagement hole
32. Radial rib
33. Cup rim
331. Reinforcing rib
34. Accommodation space
36. Locating hole
- 4.: Earmuff cushion
41. Binding strip
42. Opening
43. Fabric sleeve
- A.: Adjustable headpiece
- B.: Earmuff
- C.: Fastening member

## Claims

1. An earmuff device, comprising a headpiece (1), two earmuff cups (3), two connecting devices (2) respectively connecting said earmuff cups (3) to said headpiece (1), and two earmuff cushions (4) respectively covered on said earmuff cups (3), wherein:
said headpiece (1) is comprised of two smoothly arched bands (11) that are overlapped on each other and axially slidable relative to each other, each said band (11) having a first end, a second end opposing said first end, a through hole (13) at said first end, and a coupling block (12) disposed at said second end, an insertion slot (121) defined in said coupling block (12),
**characterized in that** two sliding grooves (14) bilaterally are extending along the length between said first end and
said second end, said first end of one said band (11) being inserted through said insertion slot (121) of the other said band (11) to have said coupling block (12) of one said band (11) be slidably coupled to said sliding grooves (14) of the other said band (11);
that each said connecting device (2) comprises a U-lug (21) attached to said first end of one said band (11) of said headpiece (1), said U-lug (21) having two through holes (211) disposed at two sides and aligned in line, a pivot member (23) fastened to said through holes (211) of said U-lug (21) and said through hole (13) of one said band (11) of said headpiece (1) to pivotally secure said U-lug (21) to said associating band (11) and a pivot housing (212) at a bottom side thereof, and a connecting strip (22) connecting said U-lug (21) to one said earmuff cup (3), said connecting strip (22) having a pivot axle (221) transversely disposed at one end thereof and pivotally coupled to said pivot housing (212) of said U-lug (21) and a connecting endpiece disposed at an opposite end thereof, said connecting endpiece (222) having at least one retaining rib (223) for engagement with one said earmuff cup (3); and
that each said earmuff cup (3) comprises a cup base (31), an insertion hole (311) defined in said cup base (31) for the insertion of said connecting strip (22) of one said connecting device (2), at least one engagement hole (312) cut through said cup base (31) and extending across said insertion hole (311) of said respective earmuff cup (3) for engagement with said at least one retaining rib (223) of said connecting endpiece (222) that is inserted into said insertion hole (311) of said respective earmuff cup (3), a gradual enlargement cup rim (33), a plurality of radial ribs (32) radially connected between said cup base (31) and said gradual enlargement cup rim (33), a plurality of smoothly arched reinforcing ribs (331) connected with said radial ribs (32), an accommodation space (34) surrounded by said radial ribs (32), said cup base (31), said cup rim (33) and said smoothly arched reinforcing ribs (331) for receiving one ear of a person wearing the earmuff device, a plurality of locating holes (36) defined between said cup rim (33) and said smoothly arched reinforcing ribs (331) and separated by said radial ribs (32).

2. The earmuff device as claimed in claim 1, **characterized in that**
said first end of each said band (11) of said headpiece (1) has a chamfered end edge (15) kept in friction engagement with said U-lug (21) of one said connecting device (2).

3. The earmuff device as claimed in claim 1, **characterized in that**
each retaining rib (223) of said connecting endpiece (222) of each said connecting device (2) has a cross section shaped like a regular triangle and defining a front guide slope (2231) and a rear upright stop edge (2232).

4. The earmuff device as claimed in claim 1, **characterized in that**
each said earmuff cushion (4) is comprised of two fabric panels respectively attached to front and back sides of one said earmuff cup (3) and a binding strip (41) stitched to said two fabric panels.

5. The earmuff device as claimed in claim 1, **characterized in that**
each said earmuff cushion (4) is made in the shape of a pocket that receives one said earmuff cup (3), said pocket being formed of two fabric panels stitched together and having an opening for the insertion of said associating earmuff cup (3) and a cuff around the opening, said cuff being turned inwards toward the inside of said pocket and inserted into one locating hole (36) of said received earmuff cup (3).

6. The earmuff device as claimed in claim 1, **characterized in that** it further comprises a soft sleeve (43) sleeved onto
said headpiece (1) and connected between said earmuff cushions (4).

## Patentansprüche

1. Ohrenschützervorrichtung, die ein Kopfstück (1), zwei Ohrenschützerschalen (3), zwei Verbindungseinrichtungen (2), die die Ohrenschützerschalen (3) an dem Kopfstück (1) befestigen, und zwei Ohrenschützerkissen (4) aufweist, die die Ohrenschützerschalen (3) bedecken, bei der:
das Kopfstück (1) aus zwei sanft gekrümmten Bändern oder Gurten (11) besteht, die einander überlappen und axial relativ zu einander verschiebbar sind, wobei jedes Band (11) ein erstes Ende, ein zweites Ende, das dem ersten Ende gegenüber steht, ein Durchgangsloch (13) an dem ersten Ende und einen Verbindungsblock (12), der an dem zweiten Ende angeordnet ist, aufweist, wobei ein Einfügeschlitz (121) in dem Verbindungsblock (12) ausgebildet ist,
**dadurch gekennzeichnet, dass** zwei Gleitnuten (14) sich beidseitig entlang der Länge zwischen dem ersten Ende und dem zweiten Ende erstrecken, wobei das erste Ende des einen Bandes (11) durch den Einfügeschlitz (121) des anderen Bandes (11) eingefügt ist, damit der Verbindungsblock (12) des einen Bandes (11) verschiebbar mit den Gleitnuten (14) des anderen Bandes (11) verbunden ist;
dass jede Verbindungseinrichtung (2) einen U-förmigen Ansatz (21), der an dem ersten Ende des einen Bandes (11) des Kopfstücks (1) angebracht ist, welcher U-förmiger Ansatz (21) zwei Durchgangslöcher (211), die an zwei Seiten angeordnet sind und in einer Linie ausgerichtet sind, wobei ein Schwenkglied (23) an den Durchgangslöchern (211) des U-förmigen Ansatzes (21) und dem Durchgangsloch (13) des einen Bandes (11) des Kopfstücks (1) befestigt ist, um den U-förmigen Ansatz (21) an dem damit verknüpften Band (11) zu befestigen, und ein Schwenkgehäuse (211) an einer Unterseite desselben aufweist, und einen Verbindungsstreifen (22) aufweist, der den U-förmigen Ansatz (21) an der einen Ohrenschützerschale (3) befestigt, welcher Verbindungsstreifen (22) eine Schwenkachse (221) aufweist, die quer an einem Ende desselben angeordnet ist und schwenkbar mit dem Schwenkgehäuse (212) des U-förmigen Ansatzes (21) und einem Verbindungsendstück verbunden ist, das an einem gegenüberliegenden Ende desselben angeordnet ist, wobei das Verbindungsendstück (222) wenigstens eine Halterippe (223) für Eingriff mit einer Ohrenschützerschale (3) hat; und
dass jede Ohrenschützerschale (3) eine Schalenbasis (31), ein Einfügeloch (311), das in der Schalenbasis (31) für Einfügung des Verbindungsstreifens (22) einer der Verbindungseinrichtungen (2) ausgebildet ist, wenigstens ein Eingriffsloch (312), das durch die Schalenbasis (31) eingeschnitten ist und sich über das Einfügeloch (311) der entsprechenden Ohrenschützerschale (3) für Eingriff mit der wenigstens einen Halterippe (223) des Verbindungsendstücks (222) erstreckt, das in das Einfügeloch (311) der entsprechenden Ohrenschützerschale (3) eingefügt ist, einen sich allmählich vergrößernden Schalenrand (33), eine Mehrzahl von radialen Rippen (32), die radial zwischen der Schalenbasis (31) und dem sich allmählich erweiternden Schalenrand (33) verbunden sind, eine Mehrzahl von sanft gekrümmten Verstärkungsrippen (331), die mit den radialen Rippen (32) verbunden sind, einen Aufnahmeraum (34), der durch die radialen Rippen (32), die Schalenbasis (31), den Schalenrand (33) und die sanft gekrümmten Verstärkungsrippen (331) umgeben ist, um ein Ohr einer Person aufzunehmen, die die Ohrenschützervorrichtung trägt, eine Mehrzahl von Lokalisierlöchern (36) aufweist, die zwischen dem Schalenrand (33) und den sanft gekrümmten Verstärkungsrippen (331) ausgebildet sind und durch die radialen Rippen (32) getrennt sind.

2. Ohrenschützervorrichtung nach Anspruch 1, **dadurch gekennzeichnet, dass**
das erste Ende jedes der Bänder (11) des Kopfstücks (1) einen abgeschrägten Endrand (15) hat, der in Reibungseingriff mit dem U-förmigen Ansatz (21) der einen Verbindungseinrichtung (2) gehalten ist.

3. Ohrenschützervorrichtung nach Anspruch 1, **dadurch gekennzeichnet, dass**
jede Halterippe (223) des Verbindungsendstücks (222) jeder Verbindungseinrichtung (2) einen Querschnitt hat, der wie ein regelmäßiges Dreieck geformt ist und eine vordere Führungsabschrägung (2231) und eine hintere aufrecht stehende Anschlagkante (2232) bildet.

4. Ohrenschützervorrichtung nach Anspruch 1, **dadurch gekennzeichnet, dass**
jedes Ohrenschützerkissen (4) aus zwei Gewebeplatten, die an Vorder- und Hinterseiten einer Ohrenschützerschale (3) angeordnet sind, und einem Verbindungsstreifen (21) besteht, der an den beiden Gewebeplatten angenäht ist.

5. Ohrenschützervorrichtung nach Anspruch 1, **dadurch gekennzeichnet, dass**
jedes Ohrenschützerkissen (4) in Form einer Tasche, die eine Ohrenschützerschale (3) aufnimmt, welche Tasche aus zwei Gewebeplatten, die zusammengenäht sind und eine Öffnung zum Einfügen der damit verknüpften Ohrenschützerschale (3) haben, und einer Manschette um die Öffnung gebildet ist, welche Manschette nach innen zur Innenseite der Tasche gewendet ist und in ein Lokalisierloch (36) der aufgenommenen Ohrenschützerschale (3) eingeführt ist.

6. Ohrenschützervorrichtung nach Anspruch 1, **dadurch gekennzeichnet, dass** sie weiter eine weiche Hülse (43) aufweist, die auf das Kopfstück (1) aufgeschoben ist und zwischen den Ohrenschützerkissen (4) verbunden ist.

## Revendications

1. Dispositif de protège-oreilles, comprenant une pièce de tête (1), deux coques de protège-oreilles (3), deux dispositifs de raccordement (2) raccordant respectivement lesdites coques de protège-oreilles (3) à ladite pièce de tête (1), et deux coussins de protège-oreilles (4) appliqués respectivement sur lesdites coques de protège-oreilles (3), dans lequel :
ladite pièce de tête (1) est composée de deux bandes légèrement arquées (11) qui se chevauchent et peuvent coulisser de manière axiale l'une par rapport à l'autre, chacune desdites bandes (11) ayant une première extrémité, une seconde extrémité opposée à ladite première extrémité, un trou de passage (13) au niveau de ladite première extrémité, et un bloc de couplage (12) disposé au niveau de ladite seconde extrémité, une fente d'insertion (121) définie dans ledit bloc de couplage (12),
**caractérisé en ce que** deux rainures coulissantes (14) s'étendent de manière bilatérale le long de la longueur entre ladite première extrémité et ladite seconde extrémité, ladite première extrémité de ladite bande (11) étant insérée à travers ladite fente d'insertion (121) de l'autre desdites bandes (11) afin d'avoir ledit bloc de couplage (12) de l'une desdites bandes (11) qui est couplé de manière coulissante auxdites rainures coulissantes (14) de l'autre desdites bandes (11) ;
**en ce que** chacun desdits dispositifs de raccordement (2) comprend une patte en forme de U (21) fixée à ladite première extrémité de ladite bande (11) de ladite pièce de tête (1), ladite patte en forme de U (21) ayant deux trous de passage (211) disposés au niveau de deux côtés et alignés, un élément de pivot (23) fixé auxdits trous de passage (211) de ladite patte en forme de U (21) et audit trou de passage (13) de ladite bande (11) de ladite pièce de tête (1) pour fixer de manière pivotante ladite patte en forme de U (21) à ladite bande associée (11) et un boîtier de pivot (212) au niveau de son côté inférieur, et une bande de raccordement (22) raccordant ladite patte en forme de U (21) à ladite coque de protège-oreilles (3), ladite bande de raccordement (22) ayant un essieu de pivot (221) disposé de manière transversale au niveau de son extrémité et couplé de manière pivotante audit boîtier de pivot (212) de ladite patte en forme de U (21) et une pièce d'extrémité de raccordement disposée au niveau de son extrémité opposée, ladite pièce d'extrémité de raccordement (222) ayant au moins une nervure de retenue (223) pour la mise en prise avec ladite coque de protège-oreilles (3) ; et
**en ce que** chacune desdites coques de protège-oreilles (3) comprend une base de coque (31), un trou d'insertion (311) défini dans ladite base de coque (31) pour l'insertion de ladite bande de raccordement (22) de l'un desdits dispositifs de raccordement (2), au moins un trou de mise en prise (312) découpé à travers ladite base de coque (31) et s'étendant sur ledit trou d'insertion (311) de ladite coque de protège-oreilles (3) respective pour la mise en prise avec ladite au moins une nervure de retenue (223) de ladite pièce d'extrémité de raccordement (222) qui est insérée dans ledit trou d'insertion (311) de ladite coque de protège-oreilles (3) respective, un bord de coque à agrandissement progressif (33), une pluralité de nervures radiales (32) radialement raccordées entre ladite base de coque (31) et ledit bord de coque à agrandissement progressif (33), une pluralité de nervures de renforcement légèrement arquées (331) raccordées avec lesdites nervures radiales (32), un espace de réception (34) entouré par lesdites nervures radiales (32), ladite base de coque (31), ledit bord de coque (33) et lesdites nervures de renforcement légèrement arquées (331) pour recevoir une oreille d'une personne qui porte le dispositif de protège-oreilles, une pluralité de trous de positionnement (36) définis entre ledit bord de coque (33) et lesdites nervures de renforcement légèrement arquées (331) et séparés par lesdites nervures radiales (32).

2. Dispositif de protège-oreilles selon la revendication 1, **caractérisé en ce que** ladite première extrémité de chacune desdites bandes (11) de ladite pièce de tête (1) a un bord d'extrémité chanfreiné (15) maintenu en mise en prise de frottement avec ladite patte en forme de U (21) de l'un desdits dispositifs de raccordement (2).

3. Dispositif de protège-oreilles selon la revendication 1, **caractérisé en ce que** chaque nervure de retenue (223) de ladite pièce d'extrémité de raccordement (222) de chacun desdits dispositifs de raccordement (2) a une section transversale formée comme un triangle régulier et définissant une inclinaison de guidage avant (2231) et un bord de butée droit arrière (2232).

4. Dispositif de protège-oreilles selon la revendication 1, **caractérisé en ce que** chacun desdits coussins de protège-oreilles (4) est composé de deux panneaux de tissu respectivement fixés sur les côtés avant et arrière de l'une desdites coques de protège-oreilles (3) et une bande de liaison (41) cousue sur lesdits deux panneaux de tissu.

5. Dispositif de protège-oreilles selon à revendication 1, **caractérisé en ce que** chacun desdits coussins de protège-oreilles (4) est réalisé selon la forme d'une poche qui reçoit ladite coque de protège-oreilles (3), ladite poche étant formée avec deux panneaux de tissu cousus ensemble et ayant une ouverture pour l'insertion de ladite coque de protège-oreilles associée (3) et une manchette autour de l'ouverture, ladite manchette étant retournée vers l'intérieur, vers l'intérieur de ladite poche et insérée dans un trou de positionnement (36) de ladite coque de protège-oreilles (3) reçue.

6. Dispositif de protège-oreilles selon la revendication 1, **caractérisé en ce qu'**il comprend en outre un manchon souple (43) emmanché sur ladite pièce de tête (1) et raccordé entre lesdits coussins de protège-oreilles (4).
